# EUROPEAN PATENT SPECIFICATION

(11) **EP 3 570 290 B1**
(45) Date of publication and mention of the grant of the patent: **02.04.2025**
(21) Application number: 18173355.1
(22) Date of filing: 18.05.2018
(51) Int. Cl.: G16H 40/63

(54) **MEDICAL APPARATUS**
MEDIZINISCHE VORRICHTUNG
APPAREIL MÉDICAL

(43) Date of publication of application: 20.11.2019
(73) Proprietor: Baxter Medical Systems GmbH + Co. KG, 07318 Saalfeld (DE)
(72) Inventor: KLEMM, Tobias, 07318 Saalfeld (DE)
(74) Representative: Reddie & Grose LLP

(56) References cited:
- US-A1- 2015 297 306
- TRUMPF MEDICAL: "TruSystem(TM) 3000 Mobile Operating Table Flexibility without compromise", BROCHURE TRUSYSTEM 3000, November 2016 (2016-11-01), pages 1 - 8, XP055517661, Retrieved from the Internet <URL:https://www.trumpfmedical.com/globalassets/pdf/brochures/en/Brochure_TruSystem_3000_EN.pdf> [retrieved on 20181022]
- CHUNG-HSIEN KUO ET AL: "Human-Oriented Design of Autonomous Navigation Assisted Robotic Wheelchair for Indoor Environments", MECHATRONICS, 2006 IEEE INTERNATIONAL CONFERENCE ON, IEEE, PI, 1 July 2006 (2006-07-01), pages 230 - 235, XP031020530, ISBN: 978-0-7803-9712-5

## Description

The invention relates to a medical apparatus, in particular to a medical apparatus comprising a basic unit and accessory components.

Heretofore, medical apparatuses consisting of a basic unit and accessory components have been known. When coupling the accessory components to the basic unit, a user had to take care about coupling of actually suitable accessory components and restrictions accompanying the coupling of specific accessory components. E.g., in a gynecological surgery, other components are used than in a neurological application.

Moreover, operating medical apparatuses become more and more complicated and, therefore, there is a risk of operating errors of the medical apparatuses, in particular, when additional accessory components are used.

In document US 2015/0297306 A1, managing of a detachable component of a medical device is shown. The medical device is formed by a pulmonary function testing system comprising a basic unit and a detachable mouthpiece having a filter. The mouthpiece comprises an identification marker which is identified by an identification module functionally associated with a controller of the pulmonary function testing system. The controller disables the pulmonary function testing system if the identification module indicates that the currently attached filter is invalid or unidentified.

The Trumpf Medical: "TruSystem(TM) 3000 Mobile Operating Table Flexibility without compromise", brochure TruSystem 3000, 1 November 2016 (2016-11-01), pages 1-8 discloses an operating table system wherein the operating table is provided with detachable components.

The independent claims of the present application differ from the prior art cited above in the features that, even though it discloses detachable components, the prior art does not disclose an accessory component comprising an information carrier including information concerning the accessory component as to interference edges of the accessory component and that the medical apparatus performs an active collision monitoring based on read-out information of the accessory component.

Therefore, the object underlying the invention is to provide a medical apparatus and a method for reliably recognizing a specific accessory component and, therefore, to significantly improve a surgical process.

The object is achieved by a medical apparatus according to claim 1 and a method according to claim 12. Advantageous further developments are subject-matter of the dependent claims. The invention is set out in the appended set of claims.

According to an aspect of the invention, a medical apparatus comprises a basic unit comprising a coupling point, an accessory component to be coupled to the coupling point of the basic unit, and a controller, wherein the accessory component comprises an information carrier including information concerning the accessory component. The basic unit comprises a sensor configured to read-out the information concerning the accessory component coupled to the basic unit from the information carrier, and the controller is configured to identify the accessory component by means of the read-out information.

By such a configuration of the medical apparatus, identifying a specific accessory component and, as the case may be, reading-out additional available information are easily and reliably possible.

In an advantageous implementation, the medical apparatus is an operating table.

Since operating tables can be equipped with a lot of different accessory components, in particular, in the case of a medical apparatus being an operating table, the identification of the accessory components is advantageous for a safe operation of the operating table.

In a further advantageous implementation, the accessory component is a patient support component.

A lot of accessory parts of an operating table are various patient support components, as, e.g., a leg section, an extension section, or a head section, so that identifying the patient support component is important for avoiding inadmissible configurations of combination of the patient support components.

According to a further advantageous implementation, the information carrier comprises a visible code.

A detection of a visible code by an optical sensor can be easily and reliably executed.

In a further advantageous implementation, the QR code is integrated in the accessory component.

By an integration of the QR code in the accessory component, a reliable attribution of the information carrier and the accessory component is possible during the entire life cycle of the accessory component.

In a further advantageous implementation, the visible code comprises a QR code.

QR codes can include a huge information content on a very small area. Therefore, less space is necessary for providing an appropriate information.

In a further advantageous implementation, the information includes at least one of a serial number, a date of manufacture, and a version of the accessory component.

By such a specification of the accessory component, a more exact determination of an update level and, therefore, of an exact configuration is possible. Thus, possible changes of the characteristics influencing the operation of the medical apparatus comprising the accessory component can be considered.

In a further advantageous implementation, the sensor is integrated in the coupling point.

By this integration, no further bracket for fixing the sensor is necessary. Furthermore, the sensor can be integrated in a safe manner so that the sensor is protected and a reliable function thereof is enabled.

In a further advantageous implementation, the sensor comprises a CMOS camera.

Due to the configuration of the sensor comprising the CMOS camera, an inexpensive optical sensor enabling a flexible use is provided.

According to the invention, the controller is configured to perform an active collision monitoring based on the read-out information.

By the active collision monitoring, positions of interference edges of the accessory component are monitored such that collisions between the accessory component and the medical apparatus or another immobile object can be prevented.

In a further advantageous implementation, the medical apparatus comprises a display device and the controller is configured to display operating instructions for a use of the accessory component and further information about the use on the display device.

By this characteristics, a safe and advanced use of the medical apparatus comprising the accessory component is possible.

In a further advantageous implementation, the controller is configured to display a step-by-step instruction about the use on the display device.

If the use of the medical apparatus comprising the accessory part is complicated, by this configuration of the medical apparatus, a safe and advanced use of the medical apparatus comprising the accessory component is possible.

According to an advantageous aspect of the invention, a method for identifying an accessory component coupled to a medical apparatus includes the steps: coupling the accessory component to the medical apparatus, reading-out the information from the information carrier of the coupled accessory component by the sensor, and identifying the accessory component by the controller.

By such a method, an identification of a specific accessory component and reading-out available information are easily and reliably possible.

According to the method of the invention, an active collision monitoring is performed based on the read-out information.

By the active collision monitoring, positions of interference edges of the accessory component are monitored such that collisions between the accessory component and the medical apparatus or another immobile object can be prevented.

In a further advantageous implementation of the method, information about the use of the accessory component are displayed on a display device.

By displaying information about the use of the accessory component, a safe and advanced use of the medical apparatus comprising the accessor component is possible. Now, the invention is elucidated by means of an embodiment referring to the drawings.

In particular,
- Fig. 1: shows an embodiment of a medical apparatus comprising accessory components;
- Fig. 2: shows an enlarged illustration of one of the accessory components and of a portion of the medical apparatus;
- Fig. 3: shows a QR code as an information carrier; and
- Fig. 4: shows a flowchart of a method according to the invention.

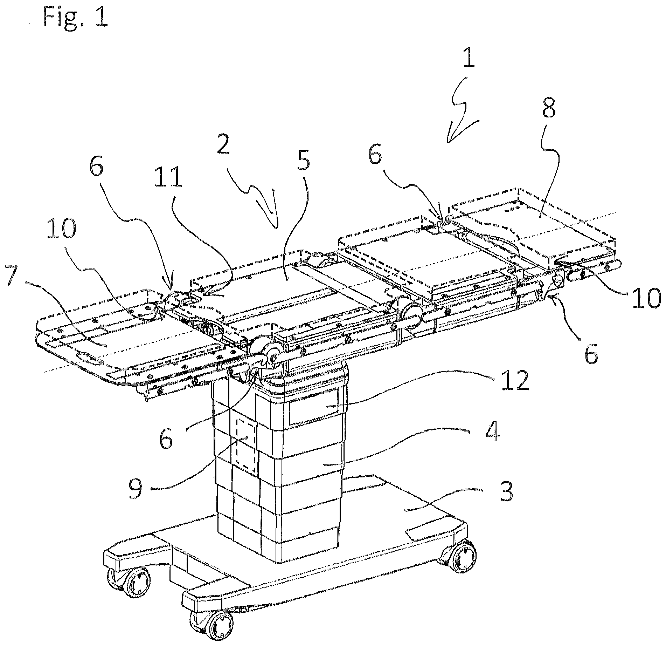

Fig. 1 shows an embodiment of a medical apparatus 1 in the form of an operating table. The operating table comprises a basic unit 2. The basic unit 2 comprises a base 3, a column 4, and a back section 5. In alternative embodiments the basic unit 2 comprises further components or less components.

In Fig. 1, two accessory components 7, 8 are illustrated. The medical apparatus 1 comprises patient support components, namely, a leg section 7 and a head section 8 as the accessory components 7, 8 to be coupled to the basic unit 2, and, therefore, to the medical apparatus 1.

The basic unit 2 comprises respectively two coupling points 6 for coupling each of the accessory components 7, 8. In an alternative embodiment, in particular, when comprising other types of accessory components, only one coupling point 6 or more than two coupling points 6 are provided for each accessory component 7, 8.

The accessory component 7, 8 comprises an information carrier 10 including information concerning the accessory component 7, 8.

The basic unit 2 comprises a sensor 11 to read-out the information concerning the accessory component 7, 8 coupled to the basic unit 2 from the information carrier 10.

The medical apparatus 1 comprises a controller 9 for controlling the functions of the medical apparatus 1. The controller 9 is configured to identify the accessory component by the read-out information. The controller 9 is further configured to perform an active collision monitoring based on the read-out information.

Furthermore, the medical apparatus 1 comprises a display device 12. The controller 9 is connected to the display device 12. Moreover, the controller 9 is configured to display operating instructions for a use of the accessory component 7, 8 and further information about the use on the display device 12. The further information are, e.g., an admissible load of the accessory component 7, 8 or an operating range. In a specific embodiment, the controller 9 is configured to display a step-by-step instruction about the use of the accessory component 7, 8 on the display device 12.

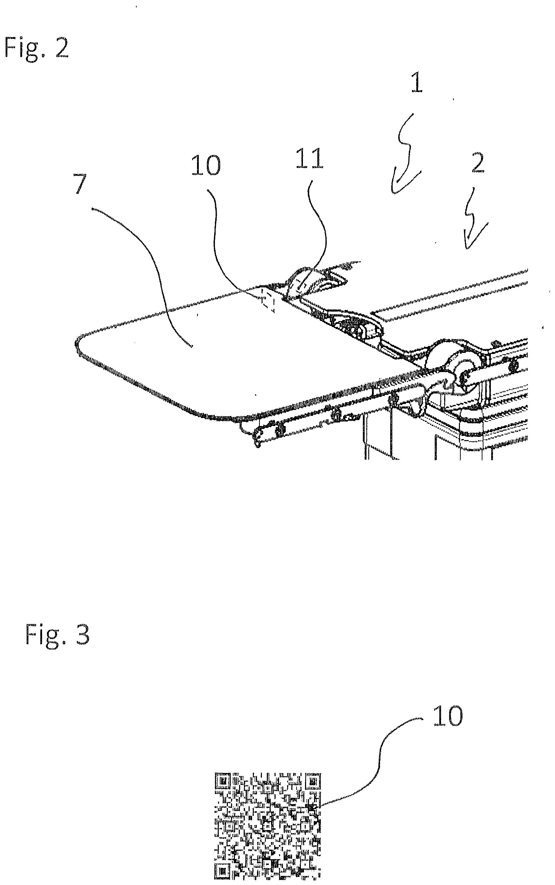

Fig. 2 shows an enlarged illustration of the accessory component 7 and of a portion of the basic unit 2 of the medical apparatus 1.

The information carrier 10 comprises a visible code. In an alternative embodiment, the information carrier 10 is not a visible code but a radio-based code, e.g., a RFID. The visible code is integrated in the accessory component 7. This is achieved, e.g., by etching the visible code in a surface of an element of the accessory component 7. In an alternative embodiment, the visible code is provided on a separate element of the accessory component 7.

The sensor 11 is integrated in the coupling point 6 on a basic unit-side. In this embodiment, the sensor 11 comprises a CMOS camera; however, in an alternative embodiment the sensor is an optical sensor having another configuration, e.g., as a CCD-sensor, or, as suitable for the information carrier 10, the sensor provides tactile sensing.

Fig. 3 shows a QR code as a visible code of an information carrier 10. The QR code enables providing a huge amount of data. The information includes at least one of a serial number, a date of manufacture and a version. Further information, as, e.g., the load, etc. can also be provided by the information carrier 10.

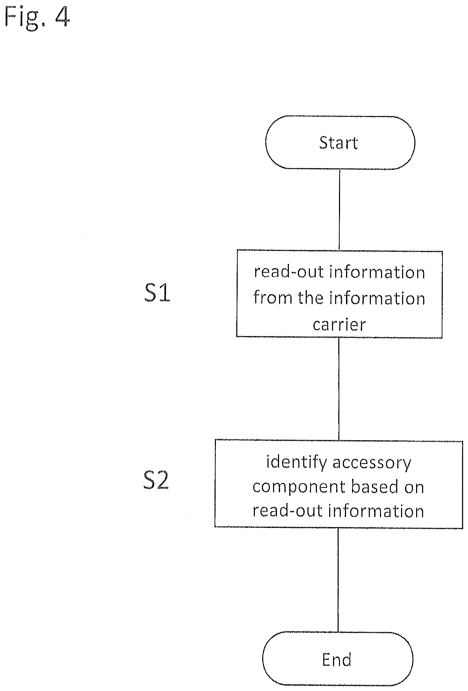

Fig. 4 shows a flowchart of a method for identifying an accessory component 7, 8 coupled to a medical apparatus 1 according to the invention. In use, if one of the accessory components 7, 8 is coupled to the basic unit, in step S1, the information is read-out from the information carrier 10 of the accessory component 7, 8 coupled to the basic unit 2 by the sensor 11. In step S2, by the controller 9, the accessory component 7, 8 is then identified base on the read-out information.

Further, the controller 9 performs an active collision monitoring based on the read-out information. Furthermore, optionally, information about the use of the accessory component 7, 8 is displayed on the display device 12.

## Claims

1. A medical apparatus (1) comprising
a basic unit (2) comprising a coupling point (6),
an accessory component (7, 8) to be coupled to the coupling point (6), and a controller (9),
wherein
the accessory component (7, 8) comprises an information carrier (10) including information concerning the accessory component (7, 8),
the basic unit (2) comprises a sensor (11) configured to read-out the information concerning the accessory component (7, 8), coupled to the basic unit (2) from the information carrier (10), and
the controller (9) is configured to identify the accessory component (7, 8) by the read-out information,
**characterized in that**
the information concerning the accessory component (7,8) includes information as to interference edges of the accessory component(7,8), and **in that**
the controller (9) is configured to perform an active collision monitoring based on the read-out information, wherein interference edges of the identified accessory component (7, 8) are monitored such that collisions between the identified accessory component (7, 8) and the medical apparatus (1) or another immobile object can be prevented.

2. The medical apparatus (1) of claim 1, wherein
the medical apparatus (1) is an operating table.

3. The medical apparatus (1) of claim 2, wherein
the accessory component (7, 8) is a patient support component.

4. The medical apparatus (1) of any preceding claim, wherein
the information carrier (10) comprises a visible code.

5. The medical apparatus (1) of claim 4, wherein
the visible code is integrated in the accessory component (7, 8).

6. The medical apparatus (1) of claim 4 or 5, wherein
the visible code comprises a QR code.

7. The medical apparatus (1) of any preceding claim, wherein
the information include at least one of a serial number, a date of manufacture, and a version of the accessory component (7, 8).

8. The medical apparatus (1) of any preceding claim, wherein
the sensor (11) is integrated in the coupling point (6).

9. The medical apparatus (1) of any preceding claim, wherein
the sensor (11) comprises a CMOS camera.

10. The medical apparatus (1) of any preceding claim, wherein
the medical apparatus (1) comprises to a display device (12) and the controller (9) is configured to display operating instructions for a use of the accessory component (7, 8) and further information about the use on the display device (12).

11. The medical apparatus (1) of claim 10, wherein
the controller (9) is configured to display a step-by-step instruction about the use on the display device (12).

12. A method for identifying an accessory component (7, 8) coupled to a medical apparatus (1) according to any preceding claim including the steps:
- reading-out the information from the information carrier (10) of the coupled accessory component (7, 8), including information as to interference edges of the accessory component (7, 8), by the sensor (11);
- identifying the accessory component (7, 8) by the controller (9) by means of the read-out information; and
- performing an active collision monitoring based on the read-out information by monitoring based on the read-out information interference edges of the identified accessory component (7, 8) such that collisions between the identified accessory component (7, 8) and the medical apparatus (1) or another immobile object can be prevented.

13. The method of claim 12, including the step:
- displaying information about the use of the accessory component (7, 8) on a display device (12).

## Patentansprüche

1. Medizinische Vorrichtung (1), die umfasst:
eine Grundeinheit (2), die einen Kopplungspunkt (6) umfasst,
ein Zubehörteil (7, 8), das mit dem Kopplungspunkt (6) zu koppeln ist, und
eine Steuerung (9),
wobei
das Zubehörteil (7, 8) einen Informationsträger (10) umfasst, der das Zubehörteil (7, 8) betreffende Informationen beinhaltet,
die Grundeinheit (2) einen Sensor (11) umfasst, der zum Auslesen der Informationen, die das Zubehörteil (7, 8), das mit der Grundeinheit (2) gekoppelt ist, betreffen, aus dem Informationsträger (10) konfiguriert ist, und
die Steuerung (9) zum Identifizieren des Zubehörteils (7, 8) anhand der ausgelesenen Informationen konfiguriert ist,
**dadurch gekennzeichnet, dass**
die Informationen, die das Zubehörteil (7, 8) betreffen, Informationen hinsichtlich Störungsrändern des Zubehörteils (7, 8) beinhalten, und dadurch, dass
die Steuerung (9) zum Durchführen einer aktiven Kollisonsüberwachung auf Basis der ausgelesenen Informationen konfiguriert ist, wobei Störkanten des identifizierten Zubehörteils (7, 8) überwacht werden, so dass Kollisionen zwischen dem identifizierten Zubehörteil (7, 8) und der medizinischen Vorrichtung (1) oder einem anderen unbeweglichen Objekt verhindert werden können.

2. Medizinische Vorrichtung (1) nach Anspruch 1, wobei
die medizinische Vorrichtung (1) ein Operationstisch ist.

3. Medizinische Vorrichtung (1) nach Anspruch 2, wobei
das Zubehörteil (7, 8) ein Patientenauflageteil ist.

4. Medizinische Vorrichtung (1) nach einem der vorhergehenden Ansprüche, wobei
der Informationsträger (10) einen sichtbaren Code umfasst.

5. Medizinische Vorrichtung (1) nach Anspruch 4, wobei
der sichtbare Code in das Zubehörteil (7, 8) integriert ist.

6. Medizinische Vorrichtung (1) nach Anspruch 4 oder 5, wobei
der sichtbare Code einen QR-Code umfasst.

7. Medizinische Vorrichtung (1) nach einem der vorhergehenden Ansprüche, wobei
die Informationen mindestens eines von einer Seriennummer, einem Herstellungsdatum und einer Version des Zubehörteils (7, 8) beinhalten.

8. Medizinische Vorrichtung (1) nach einem der vorhergehenden Ansprüche, wobei
der Sensor (11) in den Kopplungspunkt (6) integriert ist.

9. Medizinische Vorrichtung (1) nach einem der vorhergehenden Ansprüche, wobei
der Sensor (11) eine CMOS-Kamera umfasst.

10. Medizinische Vorrichtung (1) nach einem der vorhergehenden Ansprüche, wobei
die medizinische Vorrichtung (1) eine Anzeigevorrichtung (12) umfasst und die Steuerung (9) zum Anzeigen von Bedienungshinweisen für einen Gebrauch des Zubehörteils (7, 8) und weitere Informationen über den Gebrauch auf der Anzeigevorrichtung (12) konfiguriert ist.

11. Medizinische Vorrichtung (1) nach Anspruch 10, wobei
die Steuerung (9) zum Anzeigen einer Schritt-für-Schritt-Anleitung über den Gebrauch auf der Anzeigevorrichtung (12) konfiguriert ist.

12. Verfahren zum Identifizieren eines Zubehörteils (7, 8), das mit einer medizinischen Vorrichtung (1) gekoppelt ist, nach einem der vorhergehenden Ansprüche, das die folgenden Schritte beinhaltet:
- Auslesen der Informationen aus dem Informationsträger (10) des gekoppelten Zubehörteils (7, 8), die Informationen hinsichtlich Störkanten des Zubehörteils (7, 8) beinhalten, durch den Sensor (11);
- Identifizieren des Zubehörteils (7, 8) durch die Steuerung (9) mithilfe der ausgelesenen Informationen; und
- Durchführen einer aktiven Kollisionsüberwachung auf Basis der ausgelesenen Informationen durch Überwachen, auf Basis der ausgelesenen Informationen, von Störkanten des identifizierten Zubehörteils (7, 8), so dass Kollisionen zwischen dem identifizierten Zubehörteil (7, 8) und der medizinischen Vorrichtung (1) oder einem anderen unbeweglichen Objekt verhindert werden können.

13. Verfahren nach Anspruch 12 mit dem Schritt:
- Anzeigen von Informationen über den Gebrauch des Zubehörteils (7, 8) auf einer Anzeigevorrichtung (12).

## Revendications

1. Appareil médical (1) comprenant
une unité de base (2) comprenant un point de couplage (6),
un composant accessoire (7, 8) à coupler au point de couplage (6), et
un contrôleur (9),
dans lequel
le composant accessoire (7, 8) comprend un support d'informations (10) comprenant des informations concernant le composant accessoire (7, 8),
l'unité de base (2) comprend un capteur (11) configuré pour lire les informations concernant le composant accessoire (7, 8), couplé à l'unité de base (2) du support d'informations (10), et
le contrôleur (9) est configuré pour identifier le composant accessoire (7, 8) par les informations lues,
**caractérisé en ce que**
les informations concernant le composant accessoire (7, 8) comprennent des informations au sujet de bords d'interférence du composant accessoire (7, 8), et **en ce que**
le contrôleur (9) est configuré pour effectuer une surveillance active de collision sur la base des informations lues, dans lequel les bords d'interférence du composant accessoire identifié (7, 8) sont surveillés de sorte que des collisions entre le composant accessoire identifié (7, 8) et l'appareil médical (1) ou un autre objet immobile peuvent être empêchées.

2. Appareil médical (1) selon la revendication 1, où l'appareil médical (1) est une table d'opération.

3. Appareil médical (1) selon la revendication 2, dans lequel le composant accessoire (7, 8) est un composant de support de patient.

4. Appareil médical (1) selon l'une quelconque des revendications précédentes, dans lequel le support d'informations (10) comprend un code visible.

5. Appareil médical (1) selon la revendication 4, dans lequel le code visible est intégré dans le composant accessoire (7, 8).

6. Appareil médical (1) selon la revendication 4 ou 5, dans lequel le code visible comprend un code QR.

7. Appareil médical (1) selon l'une quelconque des revendications précédentes, dans lequel les informations comprennent au moins l'un d'entre un numéro de série, une date de fabrication et une version du composant accessoire (7, 8).

8. Appareil médical (1) selon l'une quelconque des revendications précédentes, dans lequel le capteur (11) est intégré dans le point de couplage (6).

9. Appareil médical (1) selon l'une quelconque des revendications précédentes, dans lequel le capteur (11) comprend une caméra CMOS.

10. Appareil médical (1) selon l'une quelconque des revendications précédentes, dans lequel l'appareil médical (1) comprend un dispositif d'affichage (12) et le contrôleur (9) est configuré pour afficher des instructions de fonctionnement pour une utilisation du composant accessoire (7, 8) et d'autres informations au sujet de l'utilisation sur le dispositif d'affichage (12).

11. Appareil médical (1) selon la revendication 10, dans lequel le contrôleur (9) est configuré pour afficher une instruction étape par étape au sujet de l'utilisation sur le dispositif d'affichage (12).

12. Procédé d'identification d'un composant accessoire (7, 8) couplé à un appareil médical (1) selon l'une quelconque des revendications précédentes comprenant les étapes consistant à :
- lire les informations du support d'informations (10) du composant accessoire couplé (7, 8), y compris des informations au sujet de bords d'interférence du composant accessoire (7, 8), par le capteur (11) ;
- identifier le composant accessoire (7, 8) par le contrôleur (9) au moyen des informations lues ; et
- effectuer une surveillance active de collision sur la base des informations lues en surveillant sur la base des informations lues des bords d'interférence du composant accessoire identifié (7, 8) de sorte que des collisions entre le composant accessoire identifié (7, 8) et l'appareil médical (1) ou un autre objet immobile peuvent être empêchées.

13. Procédé selon la revendication 12, comprenant l'étape consistant à :
- afficher des informations au sujet de l'utilisation du composant accessoire (7, 8) sur un dispositif d'affichage (12).
